# EUROPEAN PATENT APPLICATION

(11) **EP 4 796 048 A1**
(43) Date of publication of application: **26.08.2026**
(21) Application number: 26159847.8
(22) Date of filing: 20.02.2026
(51) Int. Cl.: A61B 8/08

(54) **STENT SELECTION AND DISPLAY FOR POST PERCUTANEOUS INTERVENTION INTRAVASCULAR IMAGING**

(30) Priority: 25.02.2025 US 202563763107 P
(71) Applicant: Boston Scientific Scimed, Inc., Maple Grove, Minnesota 55311 (US)
(72) Inventor: HOLMAN NARVESON, Viola Kim, Crystal, MN 55427 (US); O'BRIEN, Greta, Austin, TX 78746 (US)
(74) Representative: Vossius & Partner Patentanwälte Rechtsanwälte mbB

(57) **Abstract**

The present disclosure provides to generate a graphical user interface to visually depict stents detected from intravascular image frames and to provide for navigating between multiple detected stents via the GUI to select one of the depicted stents for interrogation. Further, the disclosure provides to update the GUI to depict assessments for the selected stent.

## Description

### CROSS-REFERENCE TO RELATED APPLICATIONS

This patent application claims the benefit of priority under 35 U.S.C. § 119 to U.S. Provisional Patent Application No. 63/763,107, filed February 25, 2025, which is herein incorporated by reference in its entirety.

### TECHNICAL FIELD

The present disclosure generally relates to intravascular imaging devices and systems arranged to capture a series of image frames and to graphical user interfaces to display indications of the captured image frames. Particularly, but not exclusively, the present disclosure relates to displaying a stent or stents represented in the captured image frames and to allowing a user to select a stent or stent segment for assessment.

### BACKGROUND

Intravascular imaging (IVI) devices are insertable into a patient's vasculature and configured to capture images from within the vessel lumen. Two common intravascular imaging modalities are intravascular ultrasound (IVUS) and optical coherence tomography (OCT). Such imaging modalities have proven diagnostic capabilities for a variety of diseases and disorders. For example, IVI devices are often used as part of a percutaneous coronary intervention (PCI) and can be used to plan, treat, and assess treatment for various obstructive coronary artery diseases, such as, unstable angina, acute myocardial infarction (MI), coronary artery disease (CAD), or the like.

As noted, an example IVI system is an IVUS system. An IVUS system includes a control module with a pulse generator, a motor drive unit, image acquisition and processing components, and a monitor. The IVUS system further includes a catheter with an ultrasound transducer included as part of the distal end of the catheter. The catheter is positioned in a lumen or cavity within, or in proximity to, a region to be imaged, such as a cardiac vessel. Often, a series of images or series of image frames are captured while the catheter is moved (e.g., pulled proximally, or the like) within the vessel lumen.

This series of image frames represent the vessel lumen structure (e.g., vessel wall, lumen, plaque, stents, etc.) along a longitudinal section of the vessel. Such images can be captured as part of a pre-PCI procedure to aid a physician in determining how to treat the patient, for example, what stent size is appropriate to treat a stenosis, stent landing zones, or the like. Further, such images can be captured as part of a post-PCI procedure to assess the results of the procedure, such as the placement and/or expansion of the stent.

However, it can be difficult for physicians to visualize the complete structure of the vessel lumen and/or the effectiveness of the PCI from the raw series if image frames. For example, it is difficult for physicians to identify key locations within the IVUS run. These key locations are often used as the basis for calculations and/or determinations related to making clinical decisions as part of a post-PCI procedure. This difficulty is compounded where multiple stents are disposed in the cardiac artery.

Thus, there here is a need for IVI systems and methods to process, annotate, visualize, and/or display images captured by IVI systems as part of a post-PCI procedure to assist physicians in making clinical decisions. Particularly, there is a need for displaying stents detected from IVI image data.

### BRIEF SUMMARY

This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter.

In general, the present disclosure provides systems and techniques to detect stents from a series of image frames captured via an IVI modality as part of a post-PCI procedure and to group the stents. Specifically, the present disclosure provides systems and techniques to present visual depictions of stents and/or stent segments for a user (e.g., physician, or the like) via a graphical user interface (GUI), where the stents and/or stent segments are detected from post-PCI IVI imaging modality runs that have a stent or stents within the imaged region. Further, the GUI provides that the user can select a stent or stent segment to assess (e.g., identification of key frames, derivation of stent expansion ratio, or the like). The GUI will be updated to reflect the selected stent and/or stent segment and the associated assessments. The present disclosure provides an improved GUI for a post-PCI workflow that allows users to quickly evaluate the stent placement and make clinical decisions to improve patient outcomes related to the post-PCI procedure.

Of note, the present disclosure provides systems and techniques to display visual representations of stents detected from a series of image frames captured via an IVI imaging modality in a graphical user interface (GUI). The detected and displayed stents can be grouped into stent segments based on the proximity of one stent to another stent. The systems and techniques further provide that a user can select a stent segment (or navigate between stent segments) via the GUI and stent assessments (e.g., detection of key frames, derivation of stent expansion ratio, or the like) can be automatically applied to the selected stent segment.

In some embodiments, the disclosure can be implemented as a computer-implemented method for an intravascular image navigation system. The computer-implemented method can comprise receiving, at a processor, a series of intravascular image (IVI) frames captured along a section of a cardiac artery of a patient where at least a first stent and a second stent are disposed in the section of the cardiac artery; receiving, at a processor, an indication of the first stent and the second stent detected from the IVI frames; generating, by the processor, a plurality of graphical indications, wherein the plurality of graphical indications comprises indications of the section of the cardiac artery based in part on the IVI frames, the first stent, and the second stent; and sending, by the processor, one or more information elements to a display to cause the display to display the graphical indications as part of a graphical user interface (GUI).

With further embodiments, the computer-implemented method comprises generating the plurality of graphical indications to further comprise indications of assessments for the first stent.

With further embodiments of the computer-implemented method, the first stent is visually depicted by the graphical indications as selected.

With further embodiments of the computer-implemented method, the first stent is selected by a user via the GUI.

With further embodiments, the computer-implemented method comprises receiving, at a processor, an indication to select the second stent; generating, by the processor, the plurality of graphical indications to further comprise indications of assessments for the second stent; and sending, by the processor, one or more additional information elements to the display to cause the display to display the graphical indications for the indications of assessments for the second stent as part of the GUI.

With further embodiments of the computer-implemented method, the second stent is visually depicted by the graphical indications as selected.

With further embodiments of the computer-implemented method, the additional information elements cause the display to not display the graphical indications for the indications of assessments for the first stent as part of the GUI.

With further embodiments of the computer-implemented method, the assessments comprise one or more key frame markers.

With further embodiments of the computer-implemented method, the one or more key frame markers comprise a distal key frame marker and a proximal key frame marker.

With further embodiments of the computer-implemented method, the one or more key frame markers comprise a minimum key frame marker.

With further embodiments of the computer-implemented method, the assessments comprise a stent expansion.

With further embodiments of the computer-implemented method, the first stent and/or the second stent is a stent segment comprising a plurality of stents.

With further embodiments of the computer-implemented method, the series of IVI frames and intravascular ultrasound (IVUS) frames.

In some embodiments, the disclosure can be implemented as an apparatus comprising a processor coupled to a memory, the memory comprising instructions executable by the processor, the processor configured to couple to an intravascular imaging (IVI) system and configured to execute the instructions which instructions when executed cause the processor to implement any of the methods described herein.

In some embodiments, the disclosure can be implemented as at least one machine readable storage device comprising a plurality of instructions that in response to being executed by a processor of an intravascular imaging (IVI) system cause the processor to implement any of the methods described herein.

In some embodiments, the disclosure can be implemented as an apparatus for an intravascular imaging system. The apparatus can comprise a processor; and a memory coupled to the processor, the memory comprising instructions executable by the processor, which instructions when executed cause the processor to receive a series of intravascular image (IVI) frames captured along a section of a cardiac artery of a patient where at least a first stent and a second stent are disposed in the section of the cardiac artery; receive an indication of the first stent and the second stent detected from the IVI frames; generate a plurality of graphical indications, wherein the plurality of graphical indications comprises indications of the section of the cardiac artery based in part on the IVI frames, the first stent, and the second stent; and send one or more information elements to a display to cause the display to display the graphical indications as part of a graphical user interface (GUI).

With some embodiments of the apparatus, the instructions, when executed by the processor further cause the processor to generate the plurality of graphical indications to further comprise indications of assessments for the first stent.

With some embodiments of the apparatus, the first stent is visually depicted by the graphical indications as selected.

With some embodiments of the apparatus, the first stent is selected by a user via the GUI.

With some embodiments of the apparatus, the instructions, when executed by the processor further cause the processor to receive an indication to select the second stent; generate the plurality of graphical indications to further comprise indications of assessments for the second stent; and send one or more additional information elements to the display to cause the display to display the graphical indications for the indications of assessments for the second stent as part of the GUI.

With some embodiments of the apparatus, the second stent is visually depicted by the graphical indications as selected.

With some embodiments of the apparatus, the additional information elements cause the display to not display the graphical indications for the indications of assessments for the first stent as part of the GUI.

With some embodiments of the apparatus, the assessments comprise one or more of a distal key frame marker, a proximal key frame marker, or a minimum key frame marker.

With some embodiments of the apparatus, the assessments comprise a stent expansion.

With some embodiments of the apparatus, the first stent and/or the second stent is a stent segment comprising a plurality of stents.

With some embodiments of the apparatus, the series of IVI frames and intravascular ultrasound (IVUS) frames.

In some embodiments, the disclosure can be implemented as at least one machine readable storage device comprising a plurality of instructions. The instructions, in response to being executed by a processor of an intravascular imaging (IVI) system cause the processor to receive a series of intravascular image (IVI) frames captured along a section of a cardiac artery of a patient where at least a first stent and a second stent are disposed in the section of the cardiac artery; receive an indication of the first stent and the second stent detected from the IVI frames; generate a plurality of graphical indications, wherein the plurality of graphical indications comprises indications of the section of the cardiac artery based in part on the IVI frames, the first stent, and the second stent; and send one or more information elements to a display to cause the display to display the graphical indications as part of a graphical user interface (GUI).

With some embodiments of the at least one machine readable storage device, the instructions when executed by the processor further cause the processor to generate the plurality of graphical indications to further comprise indications of assessments for the first stent.

With some embodiments of the at least one machine readable storage device, the instructions when executed by the processor further cause the processor to receive an indication to select the second stent; generate the plurality of graphical indications to further comprise indications of assessments for the second stent; and send one or more additional information elements to the display to cause the display to display the graphical indications for the indications of assessments for the second stent as part of the GUI, wherein the additional information elements cause the display to not display the graphical indications for the indications of assessments for the first stent as part of the GUI.

With some embodiments of the at least one machine readable storage device, the assessments comprise one or more of a distal key frame marker, a proximal key frame marker, a minimum key frame marker, or a stent expansion.

With some embodiments of the at least one machine readable storage device, the series of IVI frames and intravascular ultrasound (IVUS) frames.

### BRIEF DESCRIPTION OF THE SEVERAL VIEWS OF THE DRAWINGS

To easily identify the discussion of any element or act, the most significant digit or digits in a reference number refer to the figure number in which that element is first introduced.
FIG. 1A illustrates an example cardiac vasculature structure with a stent placed in one of the cardiac arteries and an IVUS catheter inserted into the one of the cardiac arteries.
FIG. 1B illustrates an IVUS imaging system including the IVUS catheter shown in FIG. 1A.
FIG. 2A illustrates an example series of IVUS image frames.
FIG. 2B illustrates a longitudinal representation of the example series of IVUS image frames of FIG. 2A.
FIG. 3 illustrates an IVUS navigation system.
FIG. 4A and FIG. 4B illustrate a logic flow to depict stents and/or stent segments via a graphical user interface (GUI) and to navigate through the depicted stents and/or stent segments for interrogation as part of a post-PCI procedure.
FIG. 5 illustrates a first example GUI.
FIG. 6 illustrates a second example GUI that includes the GUI of FIG. 5.
FIG. 7 illustrates a third example GUI.
FIG. 8 illustrates a fourth example GUI.
FIG. 9A illustrates a fifth example GUI.
FIG. 9B illustrates different view of the first example GUI of FIG. 9B.
FIG. 10 illustrates a computer-readable storage medium.
FIG. 11 illustrates a diagrammatic representation of a machine.

### DETAILED DESCRIPTION

The foregoing has broadly outlined the features and technical advantages of the present disclosure such that the following detailed description of the disclosure may be better understood. It is to be appreciated by those skilled in the art that the embodiments disclosed may be readily utilized as a basis for modifying or designing other structures for carrying out the same purposes of the present disclosure. The novel features of the disclosure, both as to its organization and operation, together with further objects and advantages will be better understood from the following description when considered in connection with the accompanying figures. It is to be expressly understood, however, that each of the figures is provided for the purpose of illustration and description and is not intended as a definition of the limits of the present disclosure.

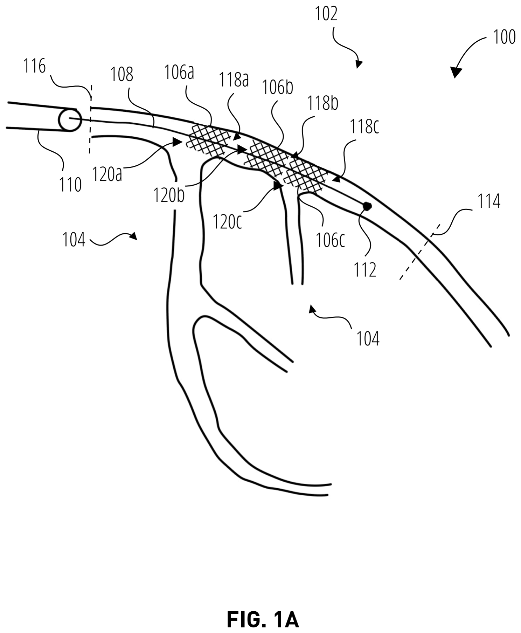

As outlined above, after some PCI procedures where a stent or stents are placed in a section of a patient's vasculature, a post-PCI procedure can be performed. The post-CI procedure is typically performed so that physicians can assess the placement of the stent(s), the expansion of the stent(s), and/or other characteristics of the PCI procedure. FIG. 1A illustrates a cardiac vasculature structure 100 having a cardiac artery 102 (e.g., cardiac artery, vessel, or the like) with several side branches 104 (e.g., descending arteries, marginal arteries, vessels, or the like) branching off the cardiac artery 102. The cardiac vasculature structure 100 further depicts stents 106a, 106b, and 106c disposed (or placed) in portions of the cardiac artery 102. It will be appreciated that the stents 106a, 106b, and/or 106c may be placed as part of a PCI procedure (or multiple procedures) to treat a stenosis in the cardiac artery 102. That is, stents 106a, 106b, and 106c can be placed as part of PCI procedure(s) (e.g., stenting balloon dilation, stenting with atherectomy, or the like) intended to dilate or open blocked or partially occluded vessels. In some examples, ones of the stents 106a, 106b, and/or 106c can be drug alluding.

As noted above, PCI procedures can be guided by intravascular imaging. For example, prior to performing a PCI procedure, during a PCI procedure, and/or after a PCI procedure; an intravascular imaging catheter (e.g., IVUS imaging catheter 108) is inserted into the cardiac artery 102 via a guide catheter 110 and images of the cardiac artery 102 are captured. The IVUS imaging catheter 108 includes an ultrasound transducer 112 disposed on a distal end of the IVUS imaging catheter 108. During the post-PCI procedure, the IVUS imaging catheter 108 can be pulled back from a distal point 114 in the cardiac artery 102 to a proximal point 116 in the cardiac artery 102 and intravascular image frames captured while the IVUS imaging catheter 108 is pulled back.

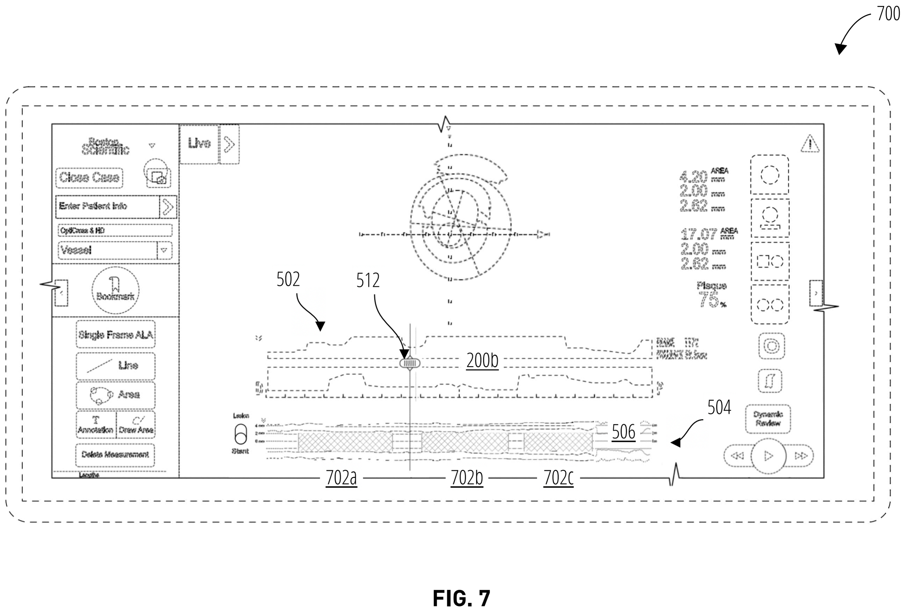

Where the PCI procedure involves placing the stents 106a, 106b, and/or 106c into the cardiac artery 102, a post-PCI procedure could involve imaging the cardiac artery 102 from the distal point 114 to the proximal point 116 to capture images of the stents 106a, 106b, and/or 106c. Physicians often use post-PCI procedures to assess how well the stents 106a, 106b, and/or 106c are placed and/or how well the stenosis of the cardiac artery 102 is corrected relative to pre-PCI images. To assist physicians in assessing the placement of the stents 106a, 106b, and/or 106c in cardiac artery 102, the present disclosure provides to group stent(s) detected from the IVUS image frames captured while the IVUS imaging catheter 108 is pulled back from distal point 114 to proximal point 116. This is explained in greater detail below. However, in general stents can grouped into stent segments based on the proximity of the stents to each other. As will be appreciated, each one of the detected stents 106a, 106b, and/or 106c will have associated edges, which can be identified. For example, stent 106a has associated stent distal edge 118a and stent proximal edge 120a, stent 106b has associated stent distal edge 118b and stent proximal edge 120b, and stent 106c has associated stent distal edge 118c and stent proximal edge 120c. The detected stents (e.g., the stents 106a, 106b, and/or 106c) can be grouped into stent segments based on the distance between adjacent stent edges. For example, stent 106a could be grouped into a first stent segment based on the distance between stent distal edge 118a and stent proximal edge 120b while the stents 106b and 106c can be grouped into a second stent segment based on the distance between stent distal edge 118b and stent proximal edge 120c (FIG. 7). With some embodiments, the distance between adjacent stent edges can be compared to a threshold distance and stents with adjacent edges less than or equal to the threshold distance grouped into the same stent segment.

The stent segments can be displayed on a GUI with each stent in each stent segment visually represented and a user can select a stent segment to interrogate via a stent assessment process. As such, a physician could utilize the provided GUI to assess the quality and/or effectiveness of the placement of stents 106a, 106b, and/or 106c in the cardiac artery 102.

It is noted that detection of stents from IVI images and grouping of stents into stent segments are outlined above is beyond the scope of this disclosure. Further, derivation of stent assessments (e.g., key frames, stent expansion, etc.) is beyond the scope of this disclosure. However, such concepts of stent detection and grouping as well as stent assessment are described in United States Provisional Patent Application No. 63/632,904 titled "Graphical User Interface for Vascular Stent Expansion Visualization" and filed on April 11, 2024; United States Provisional Patent Application No. 63/710,500 titled "Stent Detection and Grouping for Post Percutaneous Intervention Intravascular Imaging" and filed on October 22, 2024; United States Provisional Patent Application No. 63/710,510 titled "Key Frame Identification for Post Percutaneous Intervention Intravascular Imaging Based on Stent Locations" and filed on October 22, 2024; United States Patent Application Publication No. 2023/0157672, titled "Intravascular Ultrasound Imaging and Calcium Detection Methods" and filed on March 1, 2022; United States Patent Application Publication No. 2023/0112017, titled "Medical Device Systems for Automatic Lesion Assessment" and filed on October 5, 2022; United States Patent Application Publication No. 2023/0380806, titled "Systems and Methods for Intravascular Visualization" and filed on May 26, 2023; United States Patent Application Publication No. 2024/0081781, titled "Graphical User Interface for Intravascular Ultrasound Stent Display" and filed on September 13, 2023; which applications are each incorporated herein by reference in their entirety.

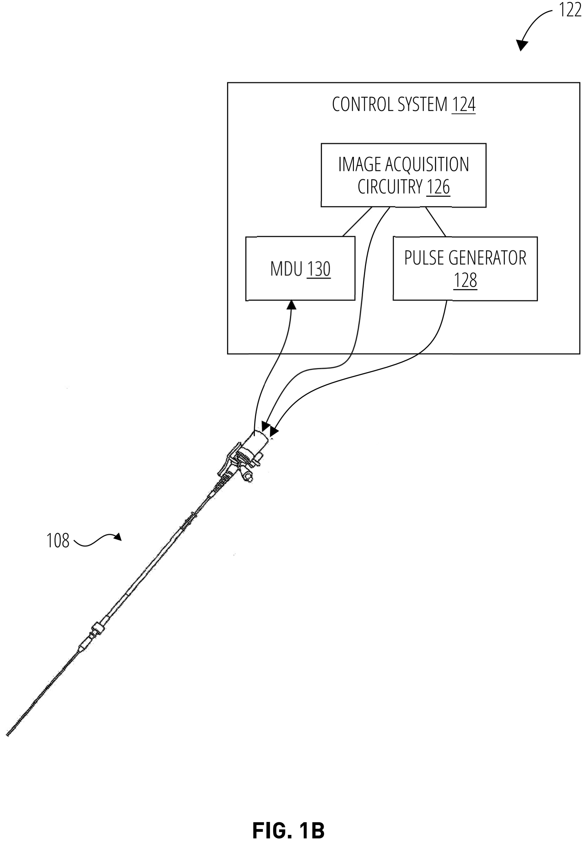

The present disclosure can be implemented for any intravascular imaging modality. However, the balance of the disclosure uses IVUS imaging (e.g., captured via IVUS imaging catheter 108, or the like) as an exemplary modality. This is done for ease of discussion only. To aid this discussion, FIG. 1B illustrates an IVUS imaging system 122. The IVUS imaging system 122 is depicted including IVUS imaging catheter 108, which can be coupled to a control system 124. The control system 124 may include, for example, an image acquisition circuitry 126, a pulse generator 128, and a motor drive unit 130. In at least some embodiments, the pulse generator 128 forms electric pulses that may be input to one or more transducers (e.g., ultrasound transducer 112, or the like) disposed on IVUS imaging catheter 108.

With some embodiments, ultrasound transducer 112 can be part of an imaging core and coupled to the proximal end of IVUS imaging catheter 108 via a drive cable or drive shaft. Mechanical energy from the motor drive unit 130 can be used to rotate the imaging core (and thus the ultrasound transducer 112).

The electrical pulses generated by pulse generator 128 can be delivered to the ultrasound transducer 112 while the ultrasound transducer 112 is rotated by the motor drive unit 130. The electrical signals are transformed by the ultrasound transducer 112 into acoustic pulses that are transmitted through the tissue of cardiac vasculature structure 100. The tissue of cardiac vasculature structure 100 reflects the acoustic pulses, which are absorbed by the ultrasound transducer 112 and transformed into electric pulses. The transformed electric pulses are delivered to the image acquisition circuitry 126 and converted into IVUS images displayable on a monitor.

For example, image acquisition circuitry 126 can be configured to map scan line samples (e.g., radial scan line samples, or the like) to a two-dimensional Cartesian grid, which can be used as the basis for a series of IVUS images that can be displayed for a user. In at least some embodiments, the image acquisition circuitry 126 may also be used to control the functioning of one or more of the other components of the control system 124. For example, the image acquisition circuitry 126 may be used to control at least one of the frequency or duration of the electrical pulses transmitted from the pulse generator 128, the rotation rate of the imaging core by the motor drive unit 130. Additionally, where IVUS imaging system 122 is configured for automatic pullback, the motor drive unit 130 can control the velocity and/or length of the pullback region (e.g., distal point 114 to proximal point 116, or the like).

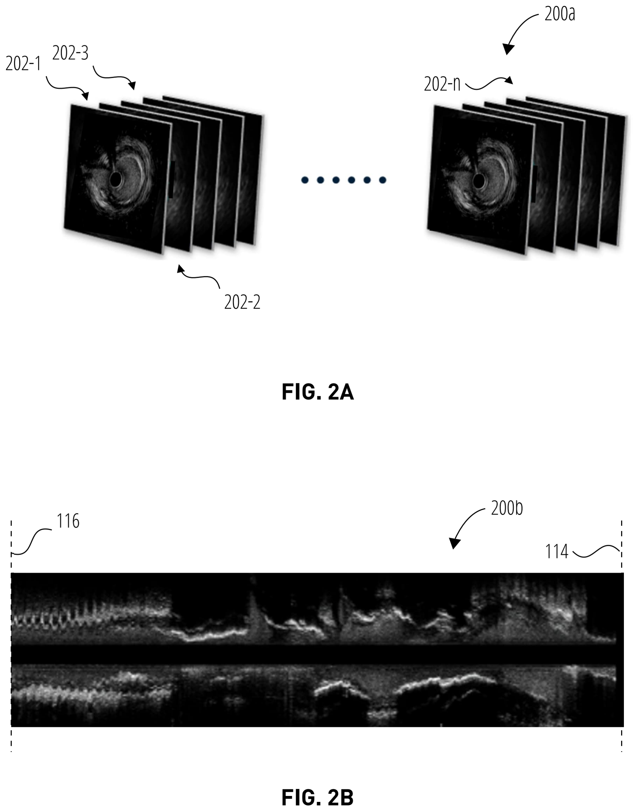

FIG. 2A and FIG. 2B illustrate a series of IVUS image frames 200a and a longitudinal view 200b of the series of IVUS image frames 200a. As described above, several IVUS image frames can be captured while IVUS imaging catheter 108 is moved through cardiac artery 102. For example, FIG. 2A depicts the series of IVUS image frames 200a including IVUS image frames 202-1, 202-2, 202-3 to 202-n, where n is a positive integer, in this case, greater than 10. In practice, n can be any positive integer greater than or equal to 2 but will often be greater than one hundred.

The series of IVUS image frames 200a can be stacked or grouped and depicted longitudinally to represent a longitudinal slice of the cardiac artery 102 from distal point 114 to proximal point 116. For example, FIG. 2B illustrates longitudinal view 200b which shows a longitudinal slice of cardiac artery 102 from distal point 114 to proximal point 116 formed from the series of IVUS image frames 200a.

As noted above, a stent or stents (e.g., stents 106a, 106b, and/or 106c) may be represented in the series of IVUS image frames 200a (e.g., where they are captured as part of a post-PCI procedure, or the like). The disclosure provides to generate graphical indications of detect stents and/or stent segments and to display the graphical indications in a GUI.

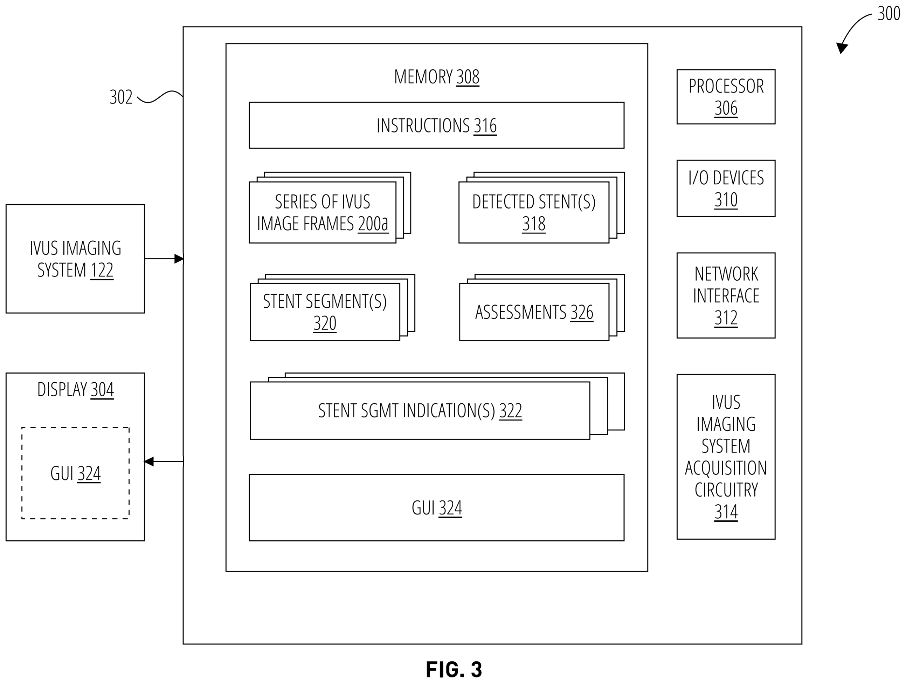

FIG. 3 illustrates an IVUS navigation system 300, which can be implemented to provide a GUI to display and allow interrogation of stents and/or stent segments detected as part of a post-PCI imaging procedure. In general, IVUS navigation system 300 is a system for processing, annotating, and/or presenting IVUS images. IVUS navigation system 300 can be implemented in a commercial IVUS guidance or navigation system, such as, for example, the AVVIGO^{™} Guidance System available from Boston Scientific^{®}. The present disclosure provides advantages over prior or conventional IVUS navigation systems in that the detected stents and/or grouped stent segments can be individually selected for interrogation (e.g., stent assessment) providing for improvements in making clinical decisions, which in turn improves patient outcomes related to the placement of stents.

IVUS navigation system 300 includes a computing device 302 and display 304. Optionally, IVUS navigation system 300 includes an IVUS imaging system, such as IVUS imaging system 122. Where IVUS navigation system 300 includes IVUS imaging system 122, IVUS navigation system 300 could be implemented as part of control system 124 or alternatively, control system 124 could be implemented as part of computing device 302 of IVUS navigation system 300. IVUS navigation system 300 will be described with reference to cardiac vasculature structure 100 and IVUS imaging system 122 of FIG. 1A and FIG. 1B as well as series of IVUS image frames 200a and longitudinal view 200b of FIG. 2A and FIG. 2B for clarity and ease of discussion. However, it is noted that IVUS navigation system 300 could be implemented for use with another IVUS imaging system or even another intravascular imaging system utilizing a different imaging modality than IVUS imaging system 122.

Computing device 302 can be any of a variety of computing devices. In some embodiments, as noted above, computing device 302 can be incorporated into and/or implemented by a console to be coupled to an intravascular imaging device (e.g., IVUS imaging system 122, IVUS imaging catheter 108, or the like). With some embodiments, computing device 302 can be a workstation or server communicatively coupled to IVUS imaging system 122. With still other embodiments, computing device 302 can be provided by a cloud-based computing device, such as, by a computing as a service system accessibly over a network (e.g., the Internet, an intranet, a wide area network, or the like). Computing device 302 can include processor 306, memory 308, input and/or output (I/O) devices 310, network interface 312, and IVUS imaging system acquisition circuitry 314.

Display 304 may include any of a variety of devices arranged to display graphical information, such as, a light emitting diode (LED) display, or the like). It is to be appreciated that although display 304 is depicted separate from computing device 302, display 304 could be implemented as part of computing device 302 or be distinct from computing device 302.

The processor 306 may include circuity or processor logic, such as, for example, any of a variety of commercial processors. In some examples, processor 306 may include multiple processors, a multi-threaded processor, a multi-core processor (whether the multiple cores coexist on the same or separate dies), and/or a multi-processor architecture of some other variety by which multiple physically separate processors are in some way linked. Additionally, in some examples, the processor 306 may include graphics processing portions and may include dedicated memory, multiple-threaded processing and/or some other parallel processing capability. In some examples, the processor 306 may be an application specific integrated circuit (ASIC) or a field programmable integrated circuit (FPGA).

The memory 308 may include logic, a portion of which includes arrays of integrated circuits, forming non-volatile memory to persistently store data or a combination of non-volatile memory and volatile memory. It is to be appreciated, that the memory 308 may be based on any of a variety of technologies. In particular, the arrays of integrated circuits included in memory 308 may be arranged to form one or more types of memory, such as, for example, dynamic random-access memory (DRAM), NAND memory, NOR memory, or the like.

I/O devices 310 can be any of a variety of devices to receive input and/or provide output. For example, I/O devices 310 can include, a keyboard, a mouse, a joystick, a foot pedal, a display, a touch enabled display, a haptic feedback device, an LED, or the like.

Network interface 312 can include logic and/or features to support a communication interface. For example, network interface 312 may include one or more interfaces that operate according to various communication protocols or standards to communicate over direct or network communication links. Direct communications may occur via use of communication protocols or standards described in one or more industry standards (including progenies and variants). For example, network interface 312 may facilitate communication over a bus, such as, for example, peripheral component interconnect express (PCIe), non-volatile memory express (NVMe), universal serial bus (USB), system management bus (SMBus), SAS (e.g., serial attached small computer system interface (SCSI)) interfaces, serial AT attachment (SATA) interfaces, or the like. Additionally, network interface 312 can include logic and/or features to enable communication over a variety of wired or wireless network standards. For example, network interface 312 may be arranged to support wired communication protocols or standards, such as, Ethernet, or the like. As another example, network interface 312 may be arranged to support wireless communication protocols or standards, such as, for example, Wi-Fi, Bluetooth, 5G, or the like.

The IVUS imaging system acquisition circuitry 314 may include circuity including custom manufactured or specially programmed circuitry configured to receive or receive and send signals with IVUS imaging system 122, including indications of intravascular images, intravascular image frames, or a series of intravascular image frames. For example, IVUS imaging system acquisition circuitry 314 can include image acquisition circuitry 126 and/or pulse generator 128.

Memory 308 can include instructions 316, series of IVUS image frames 200a, detected stent(s) 318, stent segment(s) 320, stent segment graphical indication(s) 322, graphical user interface (GUI) 324, and assessments 326.

During operation, processor 306 can execute instructions 316 to cause computing device 302 to receive (e.g., from IVUS imaging system 122, or the like) a recording of an "IVUS run" and store the recording as the series of IVUS image frames 200a in memory 308. For example, processor 306 can execute instructions 316 to receive information elements from IVUS imaging system 122 comprising indications of IVUS image frames 202-1, 202-2, 202-3 to 202-n, captured by IVUS imaging catheter 108 while IVUS imaging catheter 108 is pulled through cardiac artery 102 from distal point 114 to proximal point 116 through the stents 106a, 106b, and/or 106c. It is to be appreciated that the series of IVUS image frames 200a can be stored in a variety of image formats or even non-image formats or data structures.

The present disclosure provides to process the series of IVUS image frames 200a to both detect stent(s) from the series of IVUS image frames 200a and to group the detected stent(s) into stent segments. With some embodiments, processor 306 can execute instructions 316 to receive indications of stent(s) detected from the series of IVUS image frames 200a and store the indications as detected stent(s) 318 and to also receive indications of the of stent segment groupings based on the detected stent(s) 318 and to store the indications as stent segment(s) 320. In other embodiments, processor 306 can execute instructions 316 to detect stent(s) represented in the series of IVUS image frames 200a and store the detected stents as detected stent(s) 318 and/or to group the detected stent(s) 318 into stent segment(s) 320.

As noted, the concepts of stent detection and stent segment grouping are beyond the scope of this disclosure. However, in some embodiments, processor 306 can execute instructions 316 to identify the detected stent(s) 318 from the series of IVUS image frames 200a using machine learning (ML) models trained to identify frames of a series of frames in which a stent is represented. As another example, processor 306 can execute instructions 316 to identify the detected stent(s) 318 from the series of IVUS image frames 200a using image processing algorithms to identify stent features in frames of a series of frames. With yet another example, processor 306 can execute instructions 316 to identify the detected stent(s) 318 from the series of IVUS image frames 200a using a combination and image processing techniques and ML models. For example, processor 306 can execute instructions 316 to apply a cross-sectional segmentation to the frames of the series of IVUS image frames 200a and then infer frames representing a stent from the frame segmentations using an ML model. It is noted that processor 306 can execute instructions 316 to identify edges of the stent or stents represented in the series of IVUS image frames 200a. For example, processor 306 can execute instructions 316 to identify the frames (e.g., IVUS image frames 202-2, 202-3, etc.) where the edges of the stents 106a, 106b, and/or 106c (e.g., the stent distal edges 118a, 118b, and/or 118c and the stent proximal edges 120a, 120b, and/or 120c). Further, processor 306 can execute instructions 316 to group the detected stent(s) 318 into stent segments based on a distance between the edges of the stents (e.g., stent distal edge 118a to 118c and stent proximal edge 120a to 120c, or the like).

The present disclosure further provides to display visual indications of the detected stent(s) 318 and/or stent segment(s) 320 via a GUI. Processor 306 can execute instructions 316 to generate stent segment graphical indication(s) 322 for stent segment(s) 320 and/or generate GUI 324 from stent segment graphical indication(s) 322. In general, the stent segment graphical indication(s) 322 and/or GUI 324 can comprise visual indications of the series of IVUS image frames 200a, the detected stent(s) 318, the stent segment(s) 320, and the assessments 326 for a stent segment of the stent segment(s) 320.

In general, the stent segment graphical indication(s) 322 and/or GUI 324 can comprise visual indications of the series of IVUS image frames 200a, the detected stent(s) 318, the stent segment(s) 320. Further, the GUI 324 includes input buttons or selection methods to identify a one of the detected stent(s) 318 and/or stent segment(s) 320 to interrogate. As used herein, the term "interrogate" means to highlight the selected detected stent(s) 318 and/or stent segment(s) 320 and to derive assessments 326 for the selected detected stent(s) 318 and/or stent segment(s) 320. The GUI 324 will be updated to visually depict the derived assessments 326 for the selected detected stent(s) 318 and/or stent segment(s) 320.

As noted, derivation of stent assessments 326 is beyond the scope of this disclosure. However, in general, processor 306 can execute instructions 316 to derive the assessments 326 for each of the stent segment(s) 320. In general, the assessments 326 can include, for each stent segment of the stent segment(s) 320, vessel and/or lumen borders, raw vessel and/or lumen areas, smoothed vessel and/or lumen areas, a plaque burden, a stent expansion ratio, or the like. As another example, processor 306 can execute instructions 316 to determine the assessments (e.g., boundaries, area, plaque burden, expansion ratio, etc.) of the vessel and/or lumen depicted in each frame of the series of IVUS image frames 200a associated with each stent segment of the stent segment(s) 320. In some embodiments, this can include identifying key frames for each stent segment of the stent segment(s) 320 and then deriving the assessments 326 based on the identified key frames. With some embodiments, processor 306 can execute instructions 316 to determine the assessments 326 using image processing techniques, ML models, or a combination of image processing techniques and ML models.

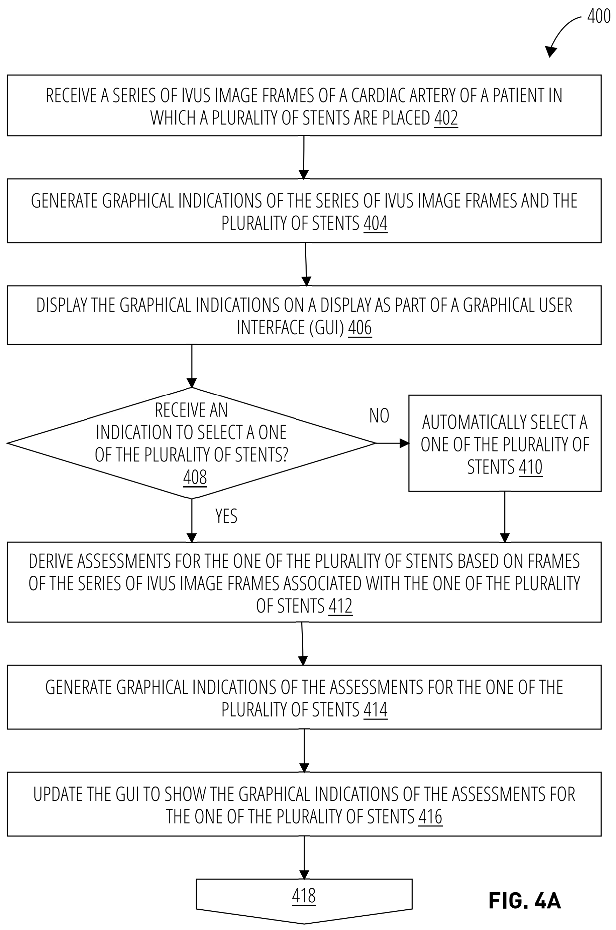

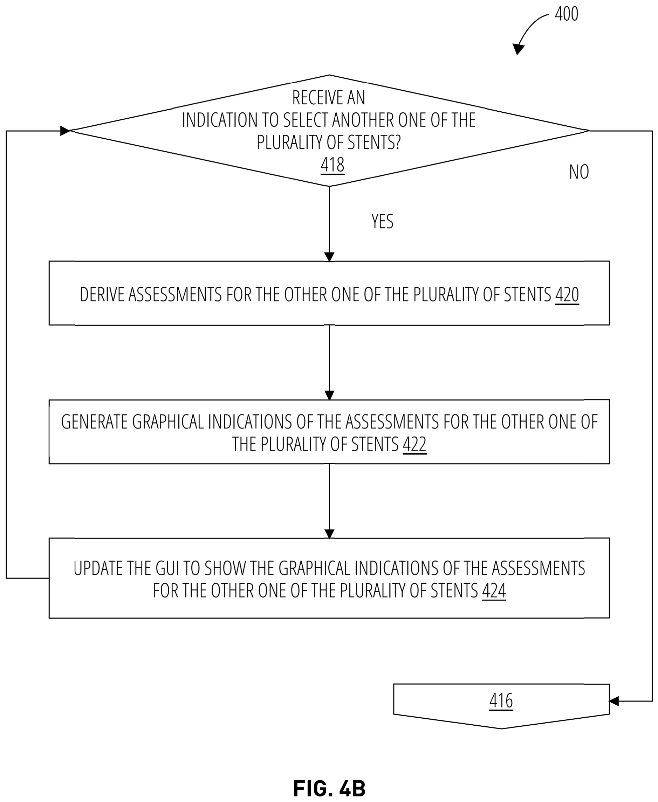

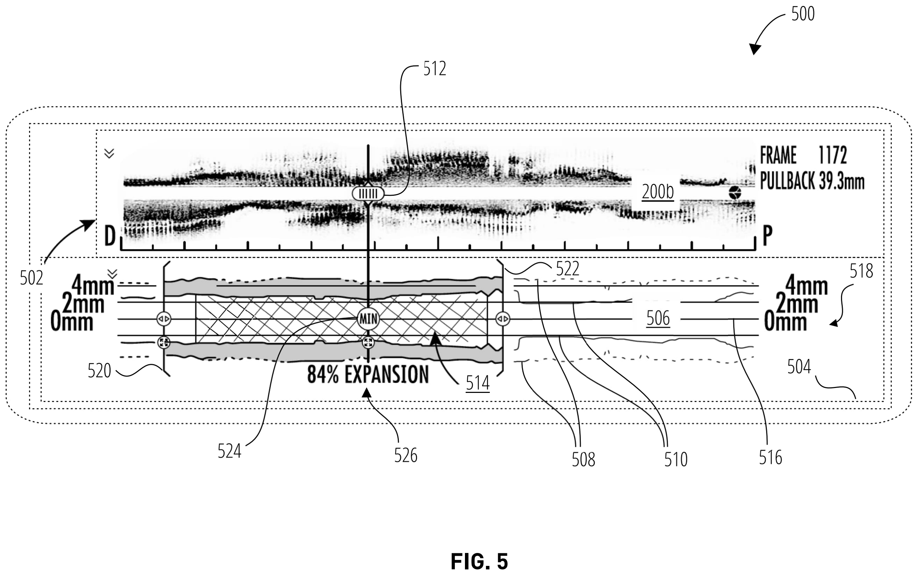

FIG. 4A and FIG. 4B illustrate a logic flow 400 to group detected stent(s) into stent segments and display visual indications of the detected stent(s) and the stent segments in a GUI. The stent(s) can be detected from a series of IVUS image frames captured during a post-PCI procedure. The logic flow 400 can be implemented by IVUS navigation system 300 and will be described with reference to IVUS navigation system 300 for clarity of presentation. However, it is noted that logic flow 400 could also be implemented by an IVUS navigation system different than IVUS navigation system 300. Further, logic flow 400 is described with reference to cardiac vasculature structure 100 and IVUS imaging system 122 of FIG. 1A and FIG. 1B and the series of IVUS image frames 200a and longitudinal view 200b of FIG. 2A and FIG. 2B for clarity of presentation. Further, logic flow 400 is described with reference to the example GUIs shown in FIG. 5, FIG. 6, FIG. 7, FIG. 8, FIG. 9A, and FIG. 9B. This is done for purposes of clarity of presentation and not limitation. That is, logic flow 400 could be implemented to generate graphical indications and GUIs different than the examples shown here.

Logic flow 400 can begin at block 402. At block 402 "receive a series of intravascular ultrasound (IVUS) image frames of a vessel of a patient in which stent(s) are placed" a series of IVUS image frames captured during a post-PCI procedure via an IVUS catheter percutaneously inserted in a vessel of a patent can be received. For example, information elements comprising indications of series of IVUS image frames 200a can be received from IVUS imaging system 122 where IVUS imaging catheter 108 is (or was) percutaneously inserted into cardiac artery 102 of cardiac vasculature structure 100. As described above, the series of IVUS image frames 200a includes several frames (e.g., IVUS image frames 202-1, 202-2, and 202-3 to 202-n) captured while the IVUS imaging catheter 108 is pulled back from the distal point 114 to proximal point 116 through the stents 106a, 106b, and/or 106c. Processor 306 can execute instructions 316 to receive information elements comprising indications of series of IVUS image frames 200a from IVUS imaging system 122, or directly from IVUS imaging catheter 108 as may be the case.

Continuing to block 404 "generate graphical indications of the series of IVUS image frames and the plurality of stents" graphical indications of the series of IVUS image frames and the detected stent(s) (and/or the stent segment(s)) can be generated. For example, processor 306 can execute instructions 316 to generate stent segment graphical indication(s) 322 comprising visual representations of series of IVUS image frames 200a, the detected stent(s) 318 and/or the stent segment(s) 320.

Continuing to block 406 "display the graphical indications on a display as part of a graphical user interface (GUI)" the graphical indications generated at block 404 can be displayed on a display as part of a GUI. For example, processor 306 can execute instructions 316 to generate GUI 324 with visual representations of the stent segment graphical indication(s) 322 and display the GUI 324 on display 304. It is to be appreciated, that in some examples, only a single stent (e.g., stent 106a, 106b, or 106c) may be detected from series of IVUS image frames 200a. Turning briefly to FIG. 5, which illustrates a GUI 500 that can be generated (e.g., at block 406 of logic flow 400, or the like) in instances where detected stent(s) 318 includes a single stent. In some examples, the GUI 500 can be generated by IVUS navigation system 300 as GUI 324 and displayed on display 304. With some embodiments, responsive to detection of detected stent(s) 318 and/or stent segment(s) 320, the processor 306 can execute instructions 316 to generate GUI 500.

GUI 500 can include a longitudinal vessel graphical indication 502 and a longitudinal vessel depiction graphical indication 504. The longitudinal vessel graphical indication 502 can include longitudinal view 200b while longitudinal vessel depiction graphical indication 504 can include a vessel profile 506 of longitudinal view 200b showing vessel borders 508 and lumen borders 510. Further, GUI 500 can include a slider 512, which can be manipulated (e.g., via I/O devices 310, or the like) to move or slide along the frames in series of IVUS image frames 200a depicted by longitudinal view 200b. Longitudinal vessel depiction graphical indication 504 can further include a depiction of the single detected stent (e.g., the stent 106a, or the like) represented in longitudinal view 200b. Longitudinal vessel depiction graphical indication 504 further depicts graphical stent indication 514 (e.g., visually representative of one of the detected stent(s) 318. With some examples, the graphical stent indication 514 can be represented as a patterned area (e.g., hatch marks as shown, mesh, screen, solid color, gradient color, or the like).

The longitudinal vessel depiction graphical indication 504 can be depicts along a longitudinal axis 516 about which the vessel profile 506 is depicted and a scale 518 measured radially out from the center of the cardiac artery 102.

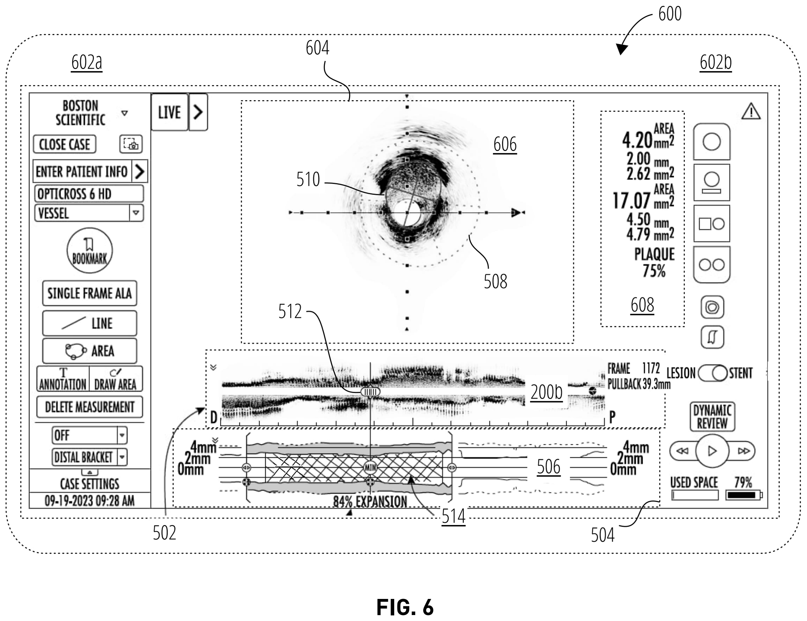

In some embodiments, a more comprehensive GUI can be generated. For example, turning briefly to FIG. 6, which illustrates a GUI 600 that can be generated by IVUS navigation system 300 as GUI 324 and displayed on display 304. It is noted that GUI 600 includes some graphical features of GUI 500. As such, GUI 600 is described with reference to theses graphical features for clarity of presentation

GUI 600 can includes menu 602a and menu 602b disposed on either sides of (or framing) graphical representations of cross-sectional frame graphical indication 604, longitudinal vessel graphical indication 502, and longitudinal vessel depiction graphical indication 504 where longitudinal vessel graphical indication 502 shows longitudinal view 200b and longitudinal vessel depiction graphical indication 504 shows vessel profile 506 and graphical stent indication 514 as described above with respect to FIG. 5 and GUI 500.

The cross-sectional frame graphical indication 604 can include depictions of on-axis IVUS image frame view 606 corresponding to the frame of series of IVUS image frames 200a in which the slider 512 is positioned. Further, cross-sectional frame graphical indication 604 can include indications of vessel border 508 and/or lumen border 510 as may be derived based on the frame depicted in on-axis IVUS image frame view 606.

In some examples, GUI 600 can include assessment graphical indication 608 showing graphical representations of assessments 326 (e.g., vessel and/or lumen area, plaque burden, etc.)

In some examples, detected stent(s) 318 can include multiple stents while stent segment(s) 320 can include one or more stent segments. Turning briefly to FIG. 7, which illustrates a GUI 700 that can be generated (e.g., at block 406 of logic flow 400, or the like) in instances where detected stent(s) 318 includes multiple stents. In some examples, the GUI 700 can be generated by IVUS navigation system 300 as GUI 324 and displayed on display 304.

GUI 700 can include features like GUIs 500 and 600 described above. For example, GUI 700 is depicted including longitudinal vessel graphical indication 502 visually depicting longitudinal view 200b, longitudinal vessel depiction graphical indication 504 visually depicting vessel profile 506 and depictions of the multiple detected stent(s) 318 (e.g., the stents 106a, 106b, and 106c). These multiple stents are depicted as graphical stent indications 702a, 702b, and 702c. Graphical stent indications 702a, 702b, and 702c can be like graphical stent indication 514 described above with respect to FIG. 5. Further, slider 512 is shown. Other features of GUI 700 are described above with respect to 500 and/or GUI 600 of FIG. 5 and/or FIG. 6.

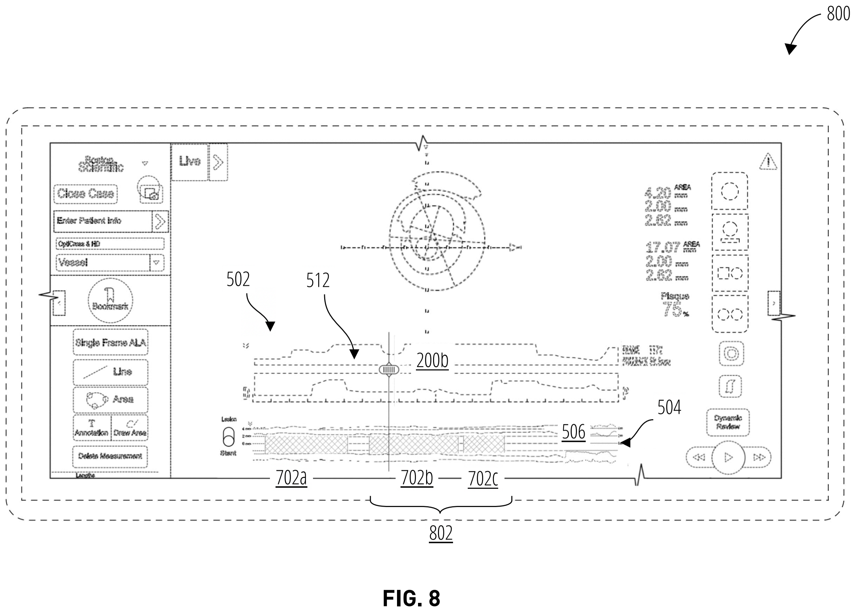

As outlined above, with some embodiments, the detected stent(s) 318 (e.g., the stents 106a, 106b, and 106c) can be grouped into stent segment(s) 320 based on the distance between adjacent stent edges. In some examples, multiple ones of the detected stent(s) 318 can be grouped into the same stent segment of stent segment(s) 320 (e.g., where adjacent stent edges of the multiple ones of the detected stent(s) 318 are less than or equal to the threshold distance away from each other). Turning briefly to FIG. 8, which illustrates a GUI 800 that can be generated (e.g., at block 406 of logic flow 400, or the like) in instances where detected stent(s) 318 includes a multiple stents and stent segment(s) 320 includes at least one stent segment. In some examples, the GUI 800 can be generated by IVUS navigation system 300 as GUI 324 and displayed on display 304.

GUI 800 can include features like GUIs 500, 600, and 700 described above. For example, GUI 800 is depicted including longitudinal vessel graphical indication 502 visually depicting longitudinal view 200b, longitudinal vessel depiction graphical indication 504 visually depicting vessel profile 506 and depictions of the multiple detected stent(s) 318 (e.g., the graphical stent indications 702a, 702b, and 702c). Additionally, longitudinal vessel depiction graphical indication 504 of GUI 800 depicted in FIG. 8 shows stents grouped into stent segment 802 (e.g., corresponding to (or including) stents associated with graphical stent indications 702b and 702c). Further, slider 512 is shown. Other features of GUI 800 are described above with respect to 500, GUI 600, and/or GUI 700 of FIG. 5, FIG. 6 and/or FIG. 7.

Returning to FIG. 4A, 400 can continue from block 406 to decision block 408. At decision block 408 "receive an indication to select a one of the plurality of stents?" a determination is made whether an indication to select a one of the plurality of stents is received. For example, processor 306 can execute instructions 316 to determine whether an indication to select one of the detected stent(s) 318 and/or stent segment(s) 320 (e.g., for interrogation, or the like) is received (e.g., via GUI 324, or the like). With some embodiments, movement of the slider 512 into a stent or stent segment may correspond to an indication to select the stent or stent segment. In other embodiments, clicking the stent or stent segment (e.g., via an input device) on the GUI 324 may correspond to an indication to select the stent or stent segment.

With some examples, the GUI 324 can visually emphasize (e.g., highlight, shade, set apart, color differently, or the like) the selected one of detected stent(s) 318 and/or stent segment(s) 320. From decision block 408, logic flow 400 can continue to block 412 or return to block 406. For example, logic flow 400 can continue from decision block 408 to block 410 or block 412. For example, logic flow 400 can continue from decision block 408 to block 412 where a determination is made that an indication to select a one of the stents or stent segment is received while logic flow 400 can continue from decision block 408 to block 410 where a determination is made that an indication to select a one of the stents or stent segments is not received.

At block 410 "automatically select a one of the plurality of stents" a one of the stents or stent segments can be automatically selected. For example, in the absence of an indication to select a particular one of detected stent(s) 318 or stent segment(s) 320 (e.g., via the GUI 324, or the like), processor 306 can execute instructions 316 to select the distal most complete stent of detected stent(s) 318 or stent segment of stent segment(s) 320 comprising the distal most complete stent of detected stent(s) 318) as the selected stent segment. With some examples, where there are no complete stent(s), processor 306 can execute instructions 316 to select the distal most stent of detected stent(s) 318 or stent segment of stent segment(s) 320 as the selected stent segment.

Logic flow 400 can continue from block 410 to block 412. At block 412 "derive assessments for the one of the plurality of stents based on frames of the series of IVUS image frames associated with the one of the plurality of stents" assessments for the selected one of the stents (or stent segments) can be derived based on the frames of the series of IVUS image frames associated with the select stent (or stent segment). For example, processor 306 can execute instructions 316 to derive assessments 326 for frames of series of IVUS image frames 200a associated with the selected stent of detected stent(s) 318 (or the stents of detected stent(s) 318 associated with the selected stent segment of stent segment(s) 320) based on a stent assessment process. In some examples, the stent assessment process can include identifying key frames associated with the stent segment, deriving vessel and/or lumen assessments (e.g., borders, area, plaque burden, etc.), and deriving stent specific assessments (e.g., stent expansion ratio, etc.)

Continuing to block 414 "generate graphical indications of the assessments for the one of the plurality of stents" graphical indications of the assessments can be generated. For example, processor 306 can execute instructions 316 to generate stent segment graphical indication(s) 322 comprising visual representations of the assessments 326 associated with the selected stent of detected stent(s) 318 (or the stents of detected stent(s) 318 associated with the selected stent segment of stent segment(s) 320).

Continuing to block 416 "update the GUI to show the graphical indications of the assessments for the one of the plurality of stents" the GUI can be updated to visually depict the graphical indications of the assessments. For example, processor 306 can execute instructions 316 to update the GUI 324 displayed on display 304 to visually depict the updated graphical indications showing assessments.

Returning to FIG. 5 and GUI 500, visual depictions of assessments 326 (e.g., key frames, or the like) are shown. For example, GUI 500 shows a distal key frame marker 520, a proximal key frame marker 522, and a minimum key frame marker 524. Additionally, the GUI 500 shows an expansion graphical indication 526 showing (e.g., as a percentage, ratio, or the like) a determined expansion of the selected stent. With some examples, the expansion ratio shown in expansion graphical indication 526 can be derived based on the minimum stent area (MSA) divided by the lumen area multiplied by 100 and visualized as a percentage as shown.

With some embodiments, processor 306 can execute instructions 316 to shade, color, or otherwise format the graphical visualization (e.g., line weight, solid line, dashed line, dotted line, area shading, area color, area pattern, etc.) to indicate plaque, confidence of border detection, a detected stent, or the like. For example, the area between vessel border 508 and lumen border 510 and between the distal key frame marker 520 and proximal key frame marker 522 can be shaded a different color than the background of GUI 500 to indicate a plaque burden of this region of the cardiac artery 102. Similarly, portions of the lines indicating the vessel border 508 and/or the lumen border 510 can be solid while other portions can be dashed indicating a confidence in the detection of the border for that respective frame of series of IVUS image frames 200a (or section of longitudinal view 200b). Similarly, vessel border 508 and lumen border 510 can be different colors to indicate which is the vessel border and which is the lumen border.

Returning to FIG. 4A, logic flow 400 can continue from block 416 to decision block 418. Decision block 418 is depicted on FIG. 4B, which shows further features of logic flow 400. At decision block 418 "receive an indication to select another one of the plurality of stents?" a determination is made whether an indication to select another one of the plurality of stents is received. For example, processor 306 can execute instructions 316 to determine whether an indication to change the selected stent of detected stent(s) 318 or stent segment of stent segment(s) 320 is received (e.g., via GUI 324, or the like).

From decision block 418, logic flow 400 can continue to block 420 or return to block 416 (shown on FIG. 4A). For example, logic flow 400 can continue from decision block 418 to block 420 where a determination is made that an indication to select another one of the plurality of stents is received while logic flow 400 can return to block 416 from decision block 418 where a determination is made at decision block 418 that an indication to select another one of the plurality of stents is not received.

At block 420 "derive assessments for the other one of the plurality of stents based on frames of the series of IVUS image frames associated with the one of the plurality of stents" assessments for the selected other one of the stents (or stent segments) can be derived based on the frames of the series of IVUS image frames associated with the select stent (or stent segment). For example, processor 306 can execute instructions 316 to derive assessments 326 for frames of series of IVUS image frames 200a associated with the newly selected other stent of detected stent(s) 318 (or the stents of detected stent(s) 318 associated with the selected other stent segment of stent segment(s) 320) based on a stent assessment process.

Continuing to block 422 "generate graphical indications of the assessments for the other one of the plurality of stents" graphical indications of the assessments can be generated. For example, processor 306 can execute instructions 316 to generate stent segment graphical indication(s) 322 comprising visual representations of the assessments 326 associated with the selected other stent of detected stent(s) 318 (or the stents of detected stent(s) 318 associated with the selected stent other segment of stent segment(s) 320).

Continuing to block 416 "update the GUI to show the graphical indications of the assessments for the other one of the plurality of stents" the GUI can be updated to visually depict the graphical indications of the assessments. For example, processor 306 can execute instructions 316 to update the GUI 324 displayed on display 304 to visually depict the updated graphical indications showing assessments for the newly selected stent (or stents of stent segment).

With some embodiments, processor 306 can execute instructions 316 to update GUI 324 to shade, color, or otherwise format the graphical depictions of the selected other stent (or stents in stent segment) as outlined above.

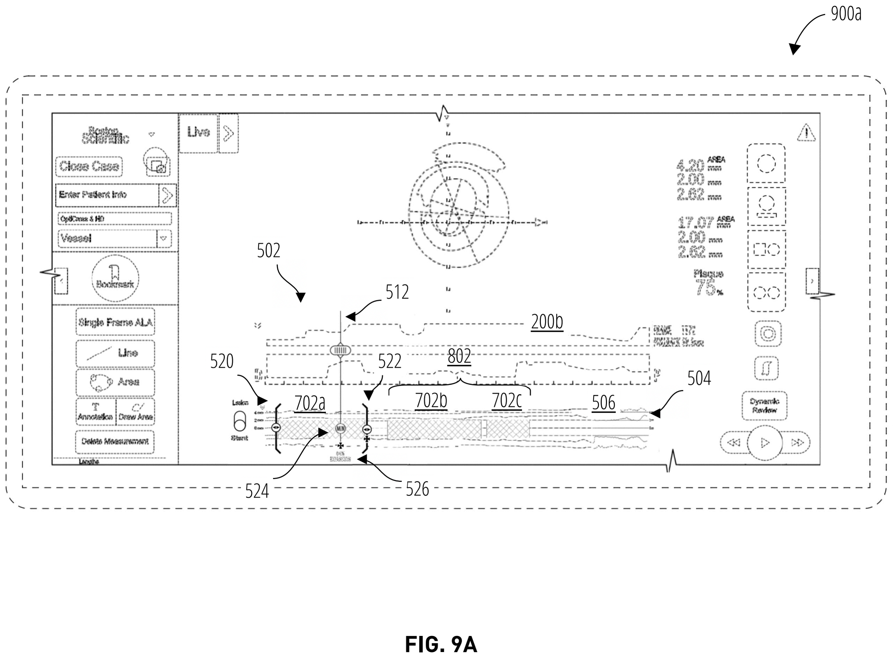

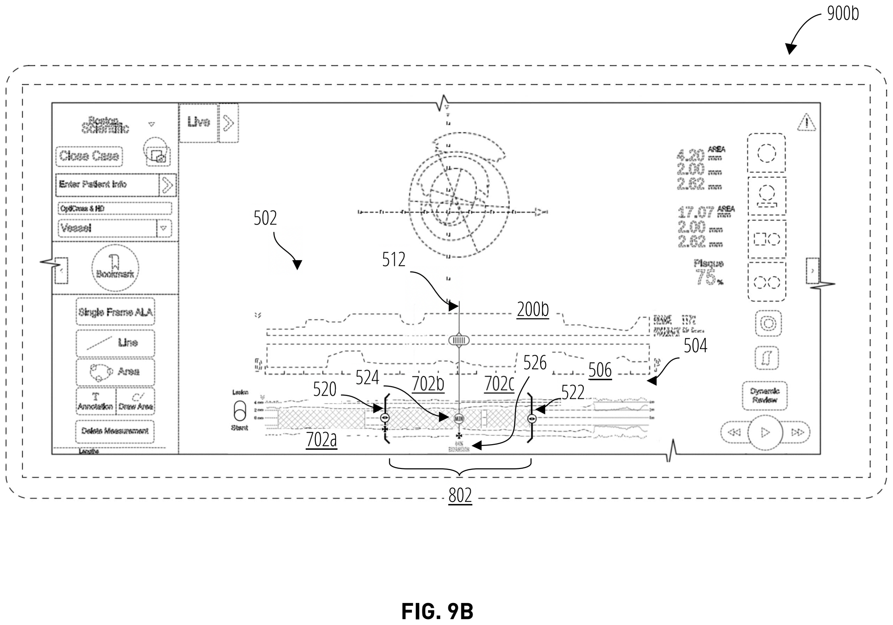

FIG. 9A and FIG. 9B illustrates an example of a GUI that is updated to visually depict selected stents and assessments for the selected stents. For example, FIG. 9A illustrates an example GUI 900a that can be generated to include indications of a selected stent (or stent segment) and assessments for the selected stent (or stent segment). Likewise, FIG. 9B illustrates an example GUI 900b can be generated (e.g., from GUI 900a, by updating GUI 900a, or the like) to include indications of another selected stent (or stent segment) and assessments for the other selected stent (or stent segment). In some embodiments, IVUS navigation system 300 can be configured to generate GUIs 900a and 900b as GUI 324 and display these GUIs on display 304. It is noted, that GUIs 900a and 900b include some graphical features of GUI 500, GUI 600, GUI 700, and/or GUI 800. As such, GUIs 900a and 900b are described with reference to theses graphical features for clarity of presentation.

GUI 900a can include longitudinal vessel graphical indication 502 visually depicting longitudinal view 200b, longitudinal vessel depiction graphical indication 504 visually depicting vessel profile 506 and depictions of the multiple detected stent(s) 318 (e.g., the graphical stent indications 702a, 702b, and 702c) and/or stent segment(s) 320 (e.g., stent segment 802). Further, slider 512 is shown. As discussed above, a one of the stents (or stent segments) can be selected (e.g., automatically or via user input) and the selected stent (or stent segment) can be emphasized and assessments 326 for the selected stent (or stent segment) can be visually depicted. As such, GUI 900a shows the stent of detected stent(s) 318 associated with graphical stent indication 702a as selected and assessments 326 for this stent visually depicted. That is, the distal key frame marker 520, the proximal key frame marker 522, the minimum key frame marker 524, and the expansion graphical indication 526 are depicted for the selected stent.

As described above, another stent (or stent segment) can be selected and the GUI 324 updated to reflect assessments for the newly selected other stent (or stent segment. FIG. 9B shows GUI 900b including longitudinal vessel graphical indication 502 visually depicting longitudinal view 200b, longitudinal vessel depiction graphical indication 504 visually depicting vessel profile 506 and depictions of the multiple detected stent(s) 318 (e.g., the graphical stent indications 702a, 702b, and 702c) and/or stent segment(s) 320 (e.g., stent segment 802). Further, slider 512 is shown.

GUI 900b shows the newly selected stent (or stent segment) of detected stent(s) 318 associated with graphical stent indication 702b and 702c as selected and assessments 326 for the stents in this stent segment visually depicted. That is, the distal key frame marker 520, the proximal key frame marker 522, the minimum key frame marker 524, and the expansion graphical indication 526 are depicted for the selected stent (or stent segment).

As can be seen, the distal key frame marker 520, the proximal key frame marker 522, the minimum key frame marker 524, and the expansion graphical indication 526 are moved to different locations than in depicted in GUI 900a.

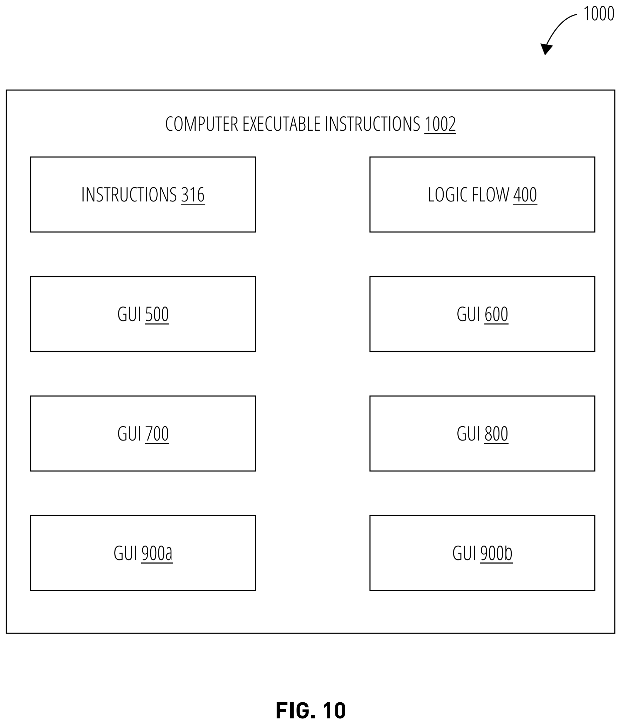

FIG. 10 illustrates computer-readable storage medium 1000. Computer-readable storage medium 1000 may comprise any non-transitory computer-readable storage medium or machine-readable storage medium, such as an optical, magnetic or semiconductor storage medium. In various embodiments, computer-readable storage medium 1000 may comprise an article of manufacture. In some embodiments, computer-readable storage medium 1000 may store computer executable instructions 1002 with which circuitry (e.g., image acquisition circuitry 126, processor 306, IVUS imaging system acquisition circuitry 314, and the like) can execute. For example, computer executable instructions 1002 can include instructions to implement operations described with respect to instructions 316, logic flow 400, GUI 500, GUI 600, GUI 700, GUI 800, GUI 900a, and/or GUI 900b. Examples of computer-readable storage medium 1000 or machine-readable storage medium may include any tangible media capable of storing electronic data, including volatile memory or non-volatile memory, removable or non-removable memory, erasable or non-erasable memory, writeable or re-writeable memory, and so forth. Examples of computer executable instructions 1002 may include any suitable type of code, such as source code, compiled code, interpreted code, executable code, static code, dynamic code, object-oriented code, visual code, and the like.

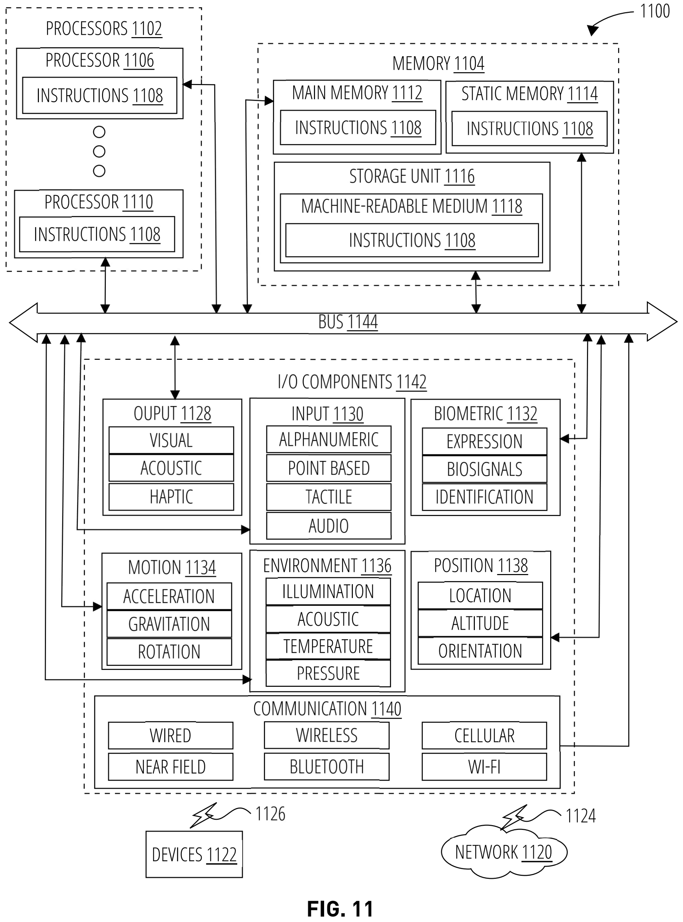

FIG. 11 illustrates a diagrammatic representation of a machine 1100 in the form of a computer system within which a set of instructions may be executed for causing the machine to perform any one or more of the methodologies discussed herein. More specifically, FIG. 11 shows a diagrammatic representation of the machine 1100 in the example form of a computer system, within which instructions 1108 (e.g., software, a program, an application, an applet, an app, or other executable code) for causing the machine 1100 to perform any one or more of the methodologies discussed herein may be executed. For example, the instructions 1108 may cause the machine 1100 to execute logic flow 400, or the like. More generally, the instructions 1108 may cause the machine 1100 to visually depict detected stents and/or stent segments and generate a GUI wherein the stent segments can be navigated through during a post-PCI procedure. It is noted that the present disclosure provides specific and discrete implementations of grouping detected stents (e.g., detected stent(s) 318) into stent segments (e.g., stent segment(s) 320) which is a significant improvement over the prior art. In particular, the present disclosure provides an improvement to computing technology in that the detected stents can be grouped and/or represented as a single segment for purposes of deriving assessments (e.g., assessments 326) and the stent(s) or stent segment(s) navigated through easily via a GUI such that assessments can be viewed and the placement of a stent as part of a post-PCI procedure evaluated to increase clinical outcomes.

The instructions 1108 transform the general, non-programmed machine 1100 into a particular machine 1100 programmed to carry out the described and illustrated functions in a specific manner. In alternative embodiments, the machine 1100 operates as a standalone device or may be coupled (e.g., networked) to other machines. In a networked deployment, the machine 1100 may operate in the capacity of a server machine or a client machine in a server-client network environment, or as a peer machine in a peer-to-peer (or distributed) network environment. The machine 1100 may comprise, but not be limited to, a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 1108, sequentially or otherwise, that specify actions to be taken by the machine 1100. Further, while a single machine 1100 is illustrated, the term "machine" shall also be taken to include several ones of machine 1100 that individually or jointly execute the instructions 1108 to perform any one or more of the methodologies discussed herein.

The machine 1100 may include processors 1102, memory 1104, and I/O components 1142, which may be configured to communicate with each other such as via a bus 1144. In an example embodiment, the processors 1102 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 1106 and a processor 1110 that may execute the instructions 1108. The term "processor" is intended to include multi-core processors that may comprise two or more independent processors (sometimes referred to as "cores") that may execute instructions contemporaneously. Although FIG. 11 shows multiple processors 1102, the machine 1100 may include a single processor with a single core, a single processor with multiple cores (e.g., a multi-core processor), multiple processors with a single core, multiple processors with multiples cores, or any combination thereof.

The memory 1104 may include a main memory 1112, a static memory 1114, and a storage unit 1116, both accessible to the processors 1102 such as via the bus 1144. The main memory 1104, the static memory 1114, and storage unit 1116 store the instructions 1108 embodying any one or more of the methodologies or functions described herein. The instructions 1108 may also reside, completely or partially, within the main memory 1112, within the static memory 1114, within machine-readable medium 1118 within the storage unit 1116, within at least one of the processors 1102 (e.g., within the processor's cache memory), or any suitable combination thereof, during execution thereof by the machine 1100.

The I/O components 1142 may include a wide variety of components to receive input, provide output, produce output, transmit information, exchange information, capture measurements, and so on. The specific I/O components 1142 that are included in a particular machine will depend on the type of machine. For example, portable machines such as mobile phones will likely include a touch input device or other such input mechanisms, while a headless server machine will likely not include such a touch input device. It will be appreciated that the I/O components 1142 may include many other components that are not shown in FIG. 11. The I/O components 1142 are grouped according to functionality merely for simplifying the following discussion and the grouping is in no way limiting. In various example embodiments, the I/O components 1142 may include output components 1128 and input components 1130. The output components 1128 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 1130 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 1142 may include biometric components 1132, motion components 1134, environmental components 1136, or position components 1138, among a wide array of other components. For example, the biometric components 1132 may include components to detect expressions (e.g., hand expressions, facial expressions, vocal expressions, body gestures, or eye tracking), measure bio signals (e.g., blood pressure, heart rate, body temperature, perspiration, or brain waves), identify a person (e.g., voice identification, retinal identification, facial identification, fingerprint identification, or electroencephalogram-based identification), and the like. The motion components 1134 may include acceleration sensor components (e.g., accelerometer), gravitation sensor components, rotation sensor components (e.g., gyroscope), and so forth. The environmental components 1136 may include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 1138 may include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1142 may include communication components 1140 operable to couple the machine 1100 to a network 1120 or devices 1122 via a coupling 1124 and a coupling 1126, respectively. For example, the communication components 1140 may include a network interface component or another suitable device to interface with the network 1120. In further examples, the communication components 1140 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 1122 may be another machine or any of a wide variety of peripheral devices (e.g., a peripheral device coupled via a USB).

Moreover, the communication components 1140 may detect identifiers or include components operable to detect identifiers. For example, the communication components 1140 may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 1140, such as location via Internet Protocol (IP) geolocation, location via Wi-Fi^{®} signal triangulation, location via detecting an NFC beacon signal that may indicate a particular location, and so forth.

The various memories (i.e., memory 1104, main memory 1112, static memory 1114, and/or memory of the processors 1102) and/or storage unit 1116 may store one or more sets of instructions and data structures (e.g., software) embodying or utilized by any one or more of the methodologies or functions described herein. These instructions (e.g., the instructions 1108), when executed by processors 1102, cause various operations to implement the disclosed embodiments.

As used herein, the terms "machine-storage medium," "device-storage medium," "computer-storage medium" mean the same thing and may be used interchangeably in this disclosure. The terms refer to a single or multiple storage devices and/or media (e.g., a centralized or distributed database, and/or associated caches and servers) that store executable instructions and/or data. The terms shall accordingly be taken to include, but not be limited to, solid-state memories, and optical and magnetic media, including memory internal or external to processors. Specific examples of machine-storage media, computer-storage media and/or device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magneto-optical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network 1120 may be an ad hoc network, an intranet, an extranet, a VPN, a LAN, a WLAN, a WAN, a WWAN, a MAN, the Internet, a portion of the Internet, a portion of the PSTN, a plain old telephone service (POTS) network, a cellular telephone network, a wireless network, a Wi-Fi^{®} network, another type of network, or a combination of two or more such networks. For example, the network 1120 or a portion of the network 1120 may include a wireless or cellular network, and the coupling 1124 may be a Code Division Multiple Access (CDMA) connection, a Global System for Mobile communications (GSM) connection, or another type of cellular or wireless coupling. In this example, the coupling 1124 may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long range protocols, or other data transfer technology.

The instructions 1108 may be transmitted or received over the network 1120 using a transmission medium via a network interface device (e.g., a network interface component included in the communication components 1140) and utilizing any one of several well-known transfer protocols (e.g., hypertext transfer protocol (HTTP)). Similarly, the instructions 1108 may be transmitted or received using a transmission medium via the coupling 1126 (e.g., a peer-to-peer coupling) to the devices 1122. The terms "transmission medium" and "signal medium" mean the same thing and may be used interchangeably in this disclosure. The terms "transmission medium" and "signal medium" shall be taken to include any intangible medium that can store or carry the instructions 1108 for execution by the machine 1100 and includes digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a matter as to encode information in the signal.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

## Claims

1. A computer-implemented method for an intravascular image navigation system, comprising:
receiving, at a processor, a series of intravascular image (IVI) frames captured along a section of a cardiac artery of a patient where at least a first stent and a second stent are disposed in the section of the cardiac artery;
receiving, at a processor, an indication of the first stent and the second stent detected from the IVI frames;
generating, by the processor, a plurality of graphical indications, wherein the plurality of graphical indications comprises indications of the section of the cardiac artery based in part on the IVI frames, the first stent, and the second stent; and
sending, by the processor, one or more information elements to a display to cause the display to display the graphical indications as part of a graphical user interface (GUI).

2. The computer-implemented method of claim 1, generating the plurality of graphical indications to further comprise indications of assessments for the first stent.

3. The computer-implemented method of claim 2, wherein the first stent is visually depicted by the graphical indications as selected.

4. The computer-implemented method of claim 3, wherein the first stent is selected by a user via the GUI.

5. The computer-implemented method of claim 2, further comprising:
receiving, at a processor, an indication to select the second stent;
generating, by the processor, the plurality of graphical indications to further comprise indications of assessments for the second stent; and
sending, by the processor, one or more additional information elements to the display to cause the display to display the graphical indications for the indications of assessments for the second stent as part of the GUI.

6. The computer-implemented method of claim 5, wherein the second stent is visually depicted by the graphical indications as selected.

7. The computer-implemented method of any one of claims 5 or 6, wherein the additional information elements cause the display to not display the graphical indications for the indications of assessments for the first stent as part of the GUI.

8. The computer-implemented method of any one of claims 2 to 7, wherein the assessments comprise one or more key frame markers.

9. The computer-implemented method of claim 8, wherein the one or more key frame markers comprise a distal key frame marker and a proximal key frame marker.

10. The computer-implemented method of any one of claims 8 or 9, wherein the one or more key frame markers comprise a minimum key frame marker.

11. The computer-implemented method of any one of claims 2 to 10, wherein the assessments comprise a stent expansion.

12. The computer-implemented method of any one of claims 1 to 11, wherein the first stent and/or the second stent is a stent segment comprising a plurality of stents.

13. The computer-implemented method of any one of claims 1 to 12, wherein the series of IVI frames and intravascular ultrasound (IVUS) frames.

14. An apparatus, comprising a processor coupled to a memory, the memory comprising instructions executable by the processor, the processor configured to couple to an intravascular imaging (IVI) system and configured to execute the instructions, which instructions when executed cause the processor to implement the method of any one of claims 1 to 13.

15. At least one machine readable storage device, comprising a plurality of instructions that in response to being executed by a processor of an intravascular imaging (IVI) system cause the processor to implement the method of any one of claim 1 to 13.
